(19) Europäisches Patentamt
European Patent Office
Office européen des brevets

(11) EP 1 080 098 B1

(12) **FASCICULE DE BREVET EUROPEEN**

(45) Date de publication et mention de la délivrance du brevet:
**09.04.2003 Bulletin 2003/15**

(21) Numéro de dépôt: **99918055.7**

(22) Date de dépôt: **11.05.1999**

(51) Int Cl.[7]: **C07F 19/00**, B01J 31/00, C07F 7/14, C07B 31/00, C07C 45/80, C08F 4/04

(86) Numéro de dépôt international:
**PCT/FR99/01128**

(87) Numéro de publication internationale:
**WO 99/060004 (25.11.1999 Gazette 1999/47)**

(54) **COMPLEXES ORGANOMETALLIQUES COMPRENANT DES CARBENES HETEROCYCLIQUES CATIONIQUES**

KATIONISCHE HETEROCYCLISCHE CARBENE ENTHALTENDE ORGANOMETALLKOMPLEXE

ORGANOMETALLIC COMPLEXES COMPRISING CATIONIC HETEROCYCLIC CARBENES

(84) Etats contractants désignés:
**BE DE ES FR GB IT NL**
Etats d'extension désignés:
**RO**

(30) Priorité: **20.05.1998 FR 9806558**

(43) Date de publication de la demande:
**07.03.2001 Bulletin 2001/10**

(73) Titulaire: **Rhodia Polyamide Intermediates**
**69192 Saint-Fons (FR)**

(72) Inventeurs:
- **BERTRAND, Guy**
  **F-31320 Pechbusque (FR)**
- **STELZIG, Lutz**
  **D-48145 Münster (DE)**
- **GUERRET, Olivier**
  **F-69280 Marcy l'Etoile (FR)**
- **BURON, Christophe**
  **F-31000 Toulouse (FR)**
- **GORNITZKA, Heinz**
  **F-31400 Toulouse (FR)**
- **BURATTIN, Paolo**
  **F-69002 Lyon (FR)**

(74) Mandataire: **Esson, Jean-Pierre et al**
**Rhodia Services,**
**Direction de la Propriété Industrielle,**
**Centre de Recherche Lyonnais,**
**BP 62**
**69192 Saint-Fons Cédex (FR)**

(56) Documents cités:
**EP-A- 0 721 951**
**EP-A- 0 721 953**
**EP-A- 0 798 041**
**WO-A-97/34875**

- **JOURNAL OF AMERICAN CHEMICAL SOCIETY vol. 119, no. 28, 1997, pages 6668 - 6669**
- **JOURNAL OF ORGANIC CHEMISTRY vol. 37, no. 14, 1972, pages 2259 - 2266**
- **JOURNAL OF CHEMICAL RESEARCH. SYNOPSES vol. 1, 1993, pages 4 - 5**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 1 080 098 B1

Printed by Jouve, 75001 PARIS (FR)

## Description

La présente invention concerne de nouveaux complexes organométalliques comprenant des carbènes hétérocycliques cationiques.

Elle concerne également, à titre de moyens pour la préparation de tels complexes organométalliques, des composés précurseurs hétérocycliques dicationiques.

Elle a encore pour objet un procédé de préparation des précédents complexes organométalliques.

Enfin elle comprend aussi l'utilisation comme catalyseurs desdits complexes organométalliques pour un certain nombre de réactions chimiques.

Des complexes organométalliques comprenant des carbènes hétérocycliques ont déjà été décrits dans l'art antérieur. Ainsi le brevet EP-A-0 721 953 décrit des complexes comportant des carbènes hétérocycliques non cationiques dérivés de l'imidazoline ou de la pyrazoline et un métal des groupes 8, 9 et 10 de la Classification périodique des éléments. Le brevet EP-A-0 798 041 décrit des complexes comportant des carbènes hétérocycliques non cationiques dérivés de différents composés ayant des cycles à 5, 6 ou 7 membres et comportant 1 ou plusieurs atomes d'azote et/ou de soufre et un métal choisi parmi le palladium, le rhodium, le nickel, le ruthénium et le cobalt. De même le brevet EP 0721951 décrit des complexes comportant des carbènes hétérocycliques non dicationiques avec des lanthanides. Le brevet WO 97/34875 décrit un procédé de fabrication de carbènes hétérocycliques non dicationiques pouvant former des complexes organométalliques. L'article paru dans J; Am; Chem. Soc. 1997, 119, 666-6669 décrit des carbènes hétérocycliques dicationiques utilisés comme précurseur de dicarbènes pour la fabrication de polymères organométalliques.

De tels complexes sont des composés très stables qui ont en outre l'avantage de ne pas former de sous-produits toxiques lors de leur dégradation. Cependant ils présentent l'inconvénient de ne pas pouvoir être utilisés en milieu trop acide et les complexes de métaux au degré d'oxydation 0 ne sont pas solubles dans l'eau, ce qui limite le champ de leur utilisation.

La présente invention concerne donc tout d'abord des complexes organométalliques comprenant des carbènes hétérocycliques, caractérisés en ce qu'ils répondent à la formule générale (I)

$$[(Z^+ X^-)_m M L_n] Y \qquad (I)$$

dans laquelle :

- $Z^+$ représente un ion 1,2,4-triazolium-5-ylidéne dont au moins une partie des atomes du cycle sont substitués par des radicaux hydrocarbonés
- L représente un ligand qui peut être ionique ou neutre
- M représente un métal choisi parmi les éléments de transition des groupes 1b, 2b, 3b, 4b, 5b, 6b, 7b et 8 de la Classification périodique des éléments telle que publiée dans "Handbook of Chemistry and Physics, 51st Edition" (1970-1971) de The Chemical Rubber Company
- $X^-$ représente un anion organique ou minéral
- m représente un nombre entier de 1 à 6
- n représente un nombre entier de 0 à 5
- la somme de m et de n est égale ou inférieure à 6
- Y représente un anion ou un cation tel que le complexe métallique soit électriquement neutre.

Par commodité on appelle complexes organométalliques ou complexes métalliques dans le présent texte, les composés de formule (I) incluant également le contre-ion Y.

Les ions 1,2,4-triazolium-5-ylidène $Z^+$ répondent à la formule générale (II) :

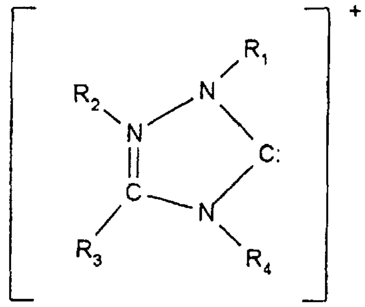

(II)

dans laquelle $R_1$, $R_2$, $R_3$ et $R_4$, identiques ou différents, représentent :

- un radical alkyle, linéaire ou ramifié, un radical cycloalkyle,
- un radical aryle,
- un radical alkyle qui comprend un ou plusieurs substituants tels qu'un radical aryle, un radical alcoxy, un atome d'halogène, un groupe hydrophile comme :
  - $COOM_1$, $-SO_3M_1$, $-PO_3M_1$, $M_1$ représentant un reste cationique minéral ou organique choisi parmi le proton, les cations dérivés des métaux alcalins ou alcalino-terreux, les cations ammonium $-N(R)_4$ dans la formule desquels les symboles R, identiques ou différents, représentent un atome d'hydrogène ou un radical alkyle ayant de 1 à 4 atomes de carbone,
  - $N(R)_3$ $Y_a$ dans la formule duquel les symboles R, identiques ou différents, représentent un atome d'hydrogène ou un radical alkyle ayant de 1 à 12 atomes de carbone, et $Y_a$ représente un anion minéral ou organique,
  - OH
- un radical aryle ou aralkyle ou cycloalkyle qui comprend sur le cycle un ou plusieurs substituants tels qu'un radical alkyle, un radical alcoxy, un atome d'halogène, un groupe hydrophile comme :
  - $COOM_1$, $-SO_3M_1$, $-PO_3M_1$, $M_1$ représentant un reste cationique minéral ou organique choisi parmi le proton, les cations dérivés des métaux alcalins ou alcalino-terreux, les cations ammonium $-N(R)_4$ dans la formule desquels les symboles R, identiques ou différents, représentent un atome d'hydrogène ou un radical alkyle ayant de 1 à 12 atomes de carbone,
  - $N(R)_3$ $Y_a$ dans la formule duquel les symboles R, identiques ou différents, représentent un atome d'hydrogène ou un radical alkyle ayant de 1 à 12 atomes de carbone, et $Y_a$ représente un anion minéral ou organique,
  - OH

$R_3$ représente également un atome d'hydrogène
$R_1$ et/ou $R_4$ peuvent aussi représenter chacun un reste ligand organique lié à l'atome d'azote de l'ion 1,2,4-triazolium-5-ylidène par un radical alkylène ou arylène, ledit reste ligand pouvant être un reste phosphite, un reste phosphonite, un reste phosphinite,
un reste phosphine ou un reste amine tertiaire aliphatique, cycloaliphatique, aromatique ou hétérocyclique et servant de ligand L au métal M.

Les restes ligands organiques précédents peuvent dériver par exemple de phosphites de phényle ou d'alkyle, substitués ou non par des substituants tels que définis précédemment, ou de phosphonites de phényle ou d'alkyle, substitués ou non par des substituants tels que définis précédemment, ou de phosphinites de phényle ou d'alkyle, substitués ou non par des substituants tels que définis précédemment, ou de phénylphosphines ou d'alkylphosphines, substituées ou non par des substituants tels que définis précédemment.

Dans un tel cas l'ion 1,2,4-triazolium-5-ylidène substitué par un radical $R_1$ et/ou $R_4$ tel que défini ci-avant est un ligand bidentate ou tridentate.

L représente un ligand ionique tel qu'un halogénure, un cyanure ou un ligand neutre tel que le monoxyde de carbone, un isonitrile, une phosphine, un phosphite organique, un phosphonate ou un phosphonite.

M représente de préférence un métal choisi parmi le nickel, le cobalt, le fer, le ruthénium, le rhodium, le palladium, l'osmium, l'iridium, le platine, le cuivre, l'argent, l'or, le zinc, le cadmium, le mercure.

Ces métaux peuvent se trouver à leurs différents degrés d'oxydation, incluant le degré d'oxydation 0.

Les anions $X^-$ sont des anions organiques ou minéraux, tels que par exemple les halogénures, mais sont

choisis de préférence parmi les anions faiblement coordinants qui sont définis par rapport au métal M considéré. Ils constituent majoritairement les contre-ions des ions 1,2,4-triazolium-5-ylidène.

A titre d'exemples non limitatifs de tels anions faiblement coordinants, on peut citer le trifluorométhylsulfonate, le tétrafluoroborate, l'hexafluorophosphate, le tétrachlorure d'aluminium, le tétrabromure d'aluminium, le tétrafluorure d'aluminium, le tétraiodure d'aluminium, le tétrachlorure de gallium, le tétrabromure de gallium, le tétrafluorure de gallium, le tétraiodure de gallium.

La présente invention a également pour objet l'utilisation de composés hétérocycliques dicationiques à titre de précurseurs pour la préparation des complexes organométalliques de formule générale (I). Ces composés hétérocycliques dicationiques répondent à la formule générale (III)

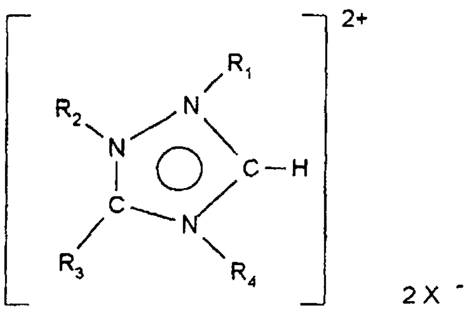

(III)

dans laquelle

- les symboles $R_1$, $R_2$, $R_3$ et $R_4$ ont les significations indiquées précédemment pour les ions 1,2,4-triazolium-5-ylidène de formule (II)
- $X^-$ représente un anion tel que ceux indiqués pour la formule générale (I).

La préparation des composés hétérocycliques dicationiques de formule générale (III) peut être réalisée en faisant réagir un composé de formule $XR_2$ dans laquelle X et $R_2$ ont les significations indiquées précédemment avec un dérivé du triazole portant les substituants $R_1$, $R_3$ et $R_4$. On peut également opérer en plusieurs étapes, chaque étape consistant à faire réagir un dérivé du triazole avec un composé de formule $XR_1$ ou $XR_3$ ou $XR_4$, selon le substituant que l'on souhaite introduire dans la formule du triazole mis en oeuvre, pour obtenir le composé hétérocyclique dicationique de formule (III). Les conditions réactionnelles peuvent être adaptées du mode opératoire décrit dans l'article de T. J. Curphey et K. S. Prasad, Journal of Org. Chem., 1972, 37, 2259.

La préparation des complexes organométalliques de formule (I) peut être réalisée en faisant réagir un composé hétérocyclique dicationique de formule générale (III) avec un composé du métal M en présence d'une base minérale ou organique. Une telle base peut être l'anion associé du composé du métal M ou être rajoutée indépendamment dans le milieu réactionnel. Cette réaction peut être effectuée en milieu liquide, généralement en ajoutant le composé du métal M et le cas échéant la base minérale ou organique à la solution ou à la suspension du composé (III). On peut bien évidemment modifier l'ordre d'introduction des différents réactifs. La réaction peut être réalisée à température ambiante ou de préférence à une température de 25°C à 150°C par exemple. Commodément cette température peut être celle du reflux du liquide dans lequel est réalisée la synthèse. La séparation du complexe de formule (I) peut être effectuée selon les méthodes utilisées habituellement en chimie, par exemple par filtration, par centrifugation ou par extraction. Les complexes de formule (I) et les composés hétérocycliques dicationiques de formule (III) sont caractérisés généralement par Résonance Magnétique Nucléaire (RMN), par Infra-rouge et par diffraction des rayons X.

Les complexes organométalliques de formule (I) peuvent servir de catalyseurs dans de nombreuses réactions chimiques.

La plupart des complexes organométalliques de formule (I) ainsi que des composés hétérocycliques dicationiques de formule (III) sont peu solubles ou insolubles dans la plupart des solvants organiques apolaires ou peu polaires comme les alcanes, les cycloalcanes, les halogénoalcanes, les hydrocarbures aromatiques ou alkylaromatiques, les éthers. Ils sont par contre solubles dans les solvants polaires. Ainsi, les composés dicationiques sont solubles notamment dans le tétrahydrofuranne, le diméthylsulfoxyde (DMSO), les nitriles (par exemple l'acétonitrile) l'eau. Ainsi, les complexes organométalliques de l'invention sont solubles notamment dans les nitriles (par exemple l'acétonitrile), le DMSO, l'eau. Ces propriétés permettent une séparation plus facile de ces différents composés, notamment par extraction liquide/liquide. D'autre part, elles permettent de mettre en oeuvre une catalyse en milieu biphasique.

A titre d'exemples non limitatifs de telles réactions, on peut citer l'hydrosilylation d'alcènes ou d'alcynes en particulier en présence de complexes du ruthénium, l'hydrosilylation des cétones en présence de complexes du ruthénium ou du rhodium, la réaction de Heck en présence de complexes du palladium, l'hydrogénation des oléfines, des aldéhydes, des acides, des énamides et des composés nitroaromatiques en présence de complexes du ruthénium, du rhodium, du platine ou du palladium, l'hydroformylation et l'hydrocarbonylation des oléfines en présence de complexes du rhodium, l'hydrocyanation des oléfines en présence de complexes du nickel, la synthèse du furanne en présence de complexes du ruthénium, la métathèse des oléfines en présence de complexes du ruthénium, la polymérisation des acrylates en présence de complexes du nickel.

EXEMPLES DE SYNTHESE DE PRECURSEURS DE FORMULE (III)

EXEMPLE 1. Synthèse du composé (IIIa) :

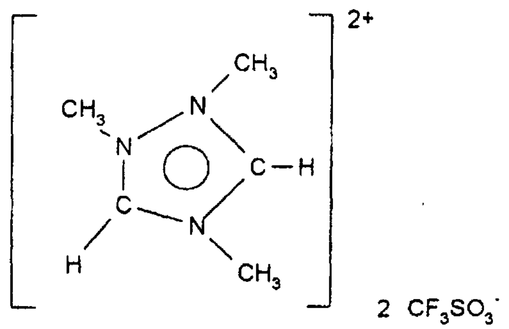

(IIIa)

En appliquant le mode opératoire décrit dans J. Org. Chem.,1972, 37, page 2259, le 1 H-1,2,4-méthyltriazole est méthylé deux fois (en positions 2 et 4 du cycle) par le trifluorométhanesulfonate de méthyle dans le 1,2-dichloroéthane à reflux sous agitation.

Le précipité brun formé est filtré, puis lavé avec du 1,2-dichloroéthane jusqu'à obtention d'une poudre blanche. Ce solide (IIIa) présente un point de fusion de 160-162°C.

Il est caractérisé par :

- RMN $^1H$ (DMSO-$d_6$) : δ 4,22 (s, 3 H, $CH_3N$) , 4,50 (s, 6 H, $CH_3N$), 10,79 (s, 2 H, $H_{cycl}$).
- RMN $^{13}C$ (H) ($CD_3CN$) : δ 38,67 (s, $CH_3N$), 38,87 (s, 2 $CH_3N$), 120,35 (q, $^1J_{CF}$ = 320,1 Hz, $CF_3$), 147,72 (s, 2 $C_{cycl}$).

EXEMPLE 2. Synthèse du composé (IIIb) :

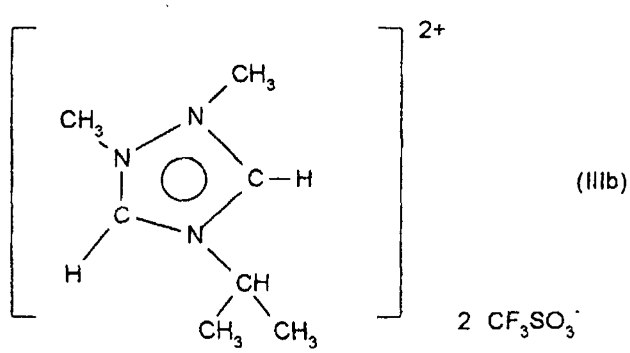

(IIIb)

La synthèse est réalisée en deux étapes. Tout d'abord un groupement isopropyle est fixé en position 4 (radical

$R_4$) par addition stoechiométrique de 1 H-1,2,4-méthyltriazole à une solution de trifluorométhanesulfonate d'isopropyle dans le 1,2-dichloroéthane (à reflux sous agitation pendant 3 h). On obtient, sous la forme d'une huile jaune, le composé de formule suivante :

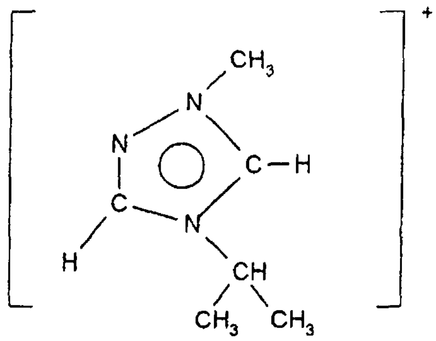

$CF_3SO_3^-$

avec un rendement de 91 % .

A une solution de 5 g (18 mmol) de ce composé dans 20 ml de 1,2-dichloroéthane, on ajoute 2,5 ml de trifluorométhanesulfonate de méthyle (22 mmol) et on porte à reflux sous agitation pendant 24 h. Il se forme un précipité brun qui est filtré, puis lavé avec du 1,2-dichloroéthane jusqu'à obtention d'une poudre blanche. Ce solide (IIIb) présente un point de fusion de 163-165°C et correspond à un rendement de 73 % par rapport au composé intermédiaire engagé.

Il est caractérisé par :

- RMN $^1H$ ($CD_3CN$) : δ 1,65 (d, $^3J_{HH}$ = 6,58 Hz, 6 H, $\underline{C}H_3CH$), 4,34 (s, 3 H, $CH_3N$), 5,03 (sept, $^3J_{HH}$ = 6,58 Hz, 1 H, $[CH_3]_2C\underline{H}N$), 10,22 (s,2 H, $H_{cycl}$).
- RMN $^{13}C$ ($CD_3CN$) : δ 21,51 (s, $\underline{C}H_3CH$), 38,71 (s, $CH_3N$), 58,88 (s, $[CH_3]_2\underline{C}HN$), 120,35 (q, $^1J_{CF}$ = 320,1 Hz, $CF_3$) ; 145,22 (s, 2 $C_{cycl}$).

<u>EXEMPLE 3</u>. Synthèse du composé (IIIc) :

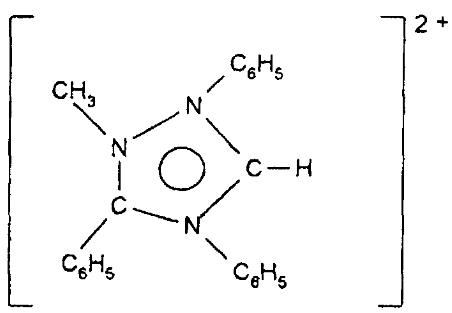

(IIIc)

2 $CF_3SO_3^-$

La synthèse est réalisée en deux étapes. Tout d'abord le composé de formulé suivante :

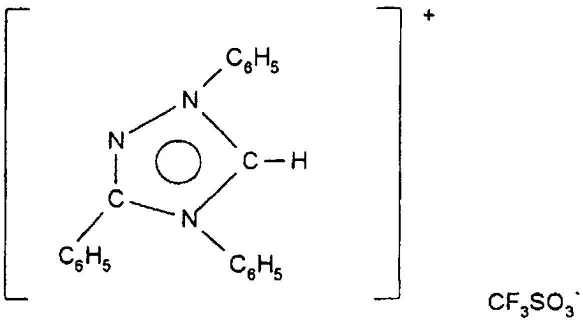

est préparé selon le mode opératoire décrit dans J. Prakt. Chem. 1988, 330, 3, 325.

Ce composé est chauffé à reflux dans le trifluorométhanesulfonate de méthyle, sous agitation pendant 4 jours. Il se forme un précipité brun qui est filtré, puis lavé avec du 1,2-dichloroéthane jusqu'à obtention d'une poudre blanche. Ce solide (IIIc) est obtenu avec un rendement de 50 % par rapport au composé engagé dans la deuxième étape.

Il est caractérisé par :

- RMN $^1H$ ($CD_3CN$) : δ 4,22 (s, 3 H, $CH_3N$), 7,4-8,4 (massif, H phényl), 10,34 (s, 1 H, $H_{cycl}$).

<u>EXEMPLE 4.</u> Synthèse du composé (IIId) :

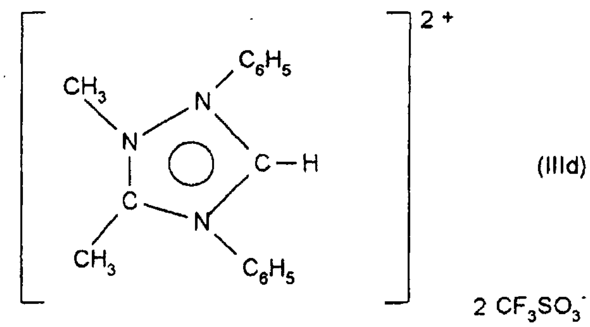

(IIId)

La synthèse est réalisée en deux étapes. Tout d'abord le composé de formule suivante :

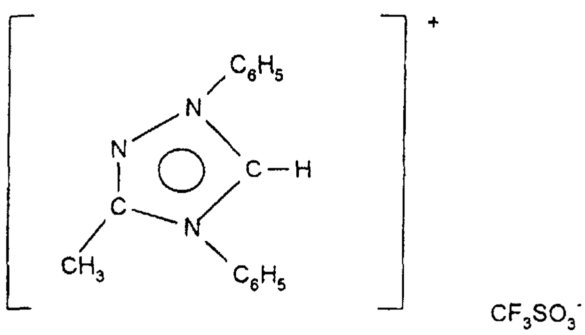

est préparé selon le mode opératoire décrit dans J. Prakt. Chem. 1988, 330, 3, 325.

Ce composé est chauffé à reflux dans le 1,2-dichloroéthane, avec 3 équivalents de trifluorométhanesulfonate de méthyle, sous agitation pendant 2 jours. Il se forme un précipité brun qui est filtré, puis lavé avec du 1,2-dichloroéthane jusqu'à obtention d'une poudre blanche. Ce solide (IIId) est obtenu avec un rendement de 69 % par rapport au composé engagé dans la deuxième étape et il présente un point de fusion de 190,5-191,3°C.

Il est caractérisé par :

- RMN $^1H$ ($CD_3CN$) : δ 3,04 (s, 3 H, $CH_3$), 4,19 (s, 3 H, $CH_3N$), 7,8-8,1 (massif, 10 H, 1 H phényl), 10,51 (s,1 H, $H_{cycl}$).
- RMN $^{13}C$ ($CD_3CN$) : δ 12,31 (s, $\underline{C}H_3C$), 38,04 (s, $CH_3N$), 126,70 (s, 2CH phényl), 128,02 (s, C phényl), 128,49 (s, 2CH phényl), 130,07 (s, C phényl), 131,56 (s, 2CH phényl), 131,83 (s, 2CH phényl), 134,04 (s, 1CH phényl), 135,88 (s, 1CH phényl), 120,35 (q, $^1J_{CF}$ = 320,1 Hz, $CF_3$), 145,58 (s, $CH_{cycl}$), 157,66 (s, $\underline{C}_{cycl}CH_3$).

Le spectre couplé proton indique une constante de couplage $^1J_{CH}$ = 240 Hz pour le CH du cycle triazole.

<u>EXEMPLE 5.</u> Synthèse du composé (IIIe) :

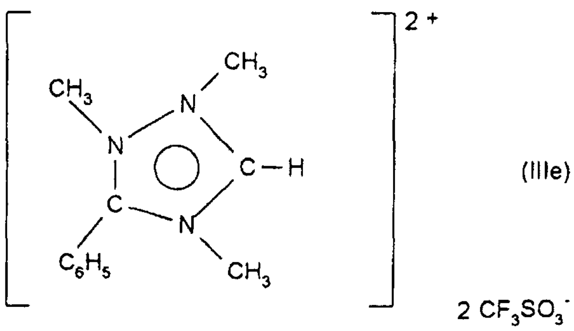

(IIIe)

La synthèse est réalisée en deux étapes. Tout d'abord le composé de formule suivante ;

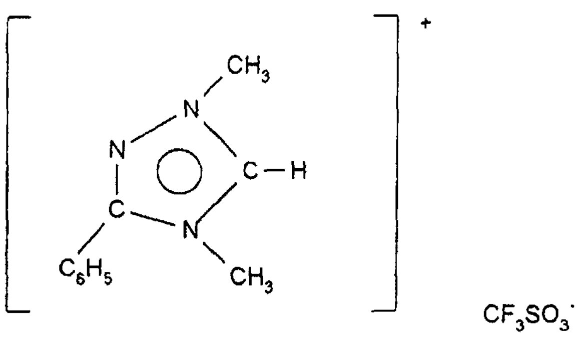

est préparé selon le mode opératoire décrit dans J. Prakt. Chem. 1988, 330, 3, 325.

Ce composé est chauffé à reflux dans le 1,2-dichloroéthane, avec 3 équivalents de trifluorométhanesulfonate de méthyle, sous agitation pendant 2 jours. Il se forme un précipité brun qui est filtré, puis lavé avec du 1,2-dichloroéthane jusqu'à obtention d'une poudre blanche. Ce solide (IIIe) est obtenu avec un rendement de 71 % par rapport au composé engagé dans la deuxième étape et il présente un point de fusion de 180,0 °C.

Il est caractérisé par :

- RMN $^1H$ ($CD_3CN$) : δ 4,08 (s, 3 H, $CH_3N$), 4,20 (s, 3 H, $CH_3N$), 4,46 (s, 3 H, $CH_3N$), 7,8-8,1 (massif, 10 H, 5 H phényl), 10,16 (s,1 H, $H_{cycl}$).
- RMN $^{13}C$ ($CD_3CN$) : δ 37,88 (s, $\underline{C}H_3N$), 38,36 (s, $CH_3N$), 39,42 (s, $CH_3N$), 130,78 (s, C phényl), 131,97 (s, 2CH phényl), 132,15 (s, 2CH phényl), 137,35 (s, CH phényl), 131,83 (s, 2CH phényl), 120,35 (q, $^1J_{CF}$ = 320,1 Hz, $CF_3$), 147,35 (s, $CH_{cycl}$), 156,48 (s, $\underline{C}_{cycl}$).

EXEMPLE 6. Synthèse du composé (IIIf) :

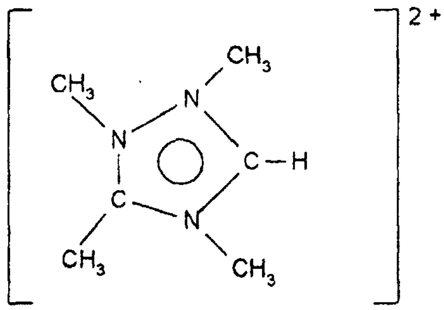

(IIIf)

2 $CF_3SO_3^-$

La synthèse est réalisée en deux étapes. Tout d'abord le composé de formule suivante :

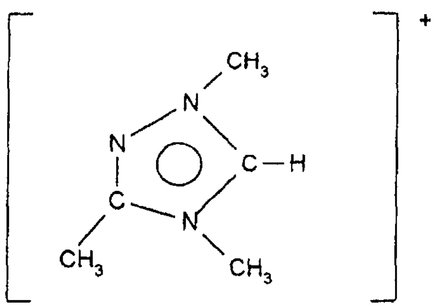

$CF_3SO_3^-$

est préparé selon le mode opératoire décrit dans J. Prakt. Chem. 1988, 330, 3, 325.

Ce composé est chauffé à reflux dans le 1,2-dichloroéthane, avec 3 équivalents de trifluorométhanesulfonate de méthyle, sous agitation pendant 2 jours. Il se forme un précipité brun qui est filtré, puis lavé avec du 1,2-dichloroéthane jusqu'à obtention d'une poudre blanche. Ce solide (IIIf) est obtenu avec un rendement de 95 % par rapport au composé engagé dans la deuxième étape et il présente un point de fusion de 194,4 °C.

Il est caractérisé par :

- RMN $^1$H (Acétone $d_6$) : δ 3,35 (s, 3 H, $CH_3C$), 4,46 (s, 3 H, $CH_3N$), 4,69 (s, 3 H, $CH_3N$), 4,77 (s, 3 H, $CH_3N$), 10,77 (s, 1 H, $H_{cycl}$).
- RMN $^{13}$C (Acétone $d_6$) : δ 10,53 (s, $\underline{C}H_3C$), 35,91 (s, $CH_3N$), 36,26 (s, $CH_3N$), 37,99 (s, $CH_3N$), 120,35 (q, $^1J_{CF}$ = 320,1 Hz, $CF_3$), 145,35 (s, $CH_{cycl}$), 157,31 (s, $\underline{C}_{cycl}CH_3$).

EXEMPLES DE SYNTHESE DES COMPLEXES ORGANOMETALLIQUES DE FORMULE (I)

EXEMPLE 7. Synthèse du composé (Ia) d'Ag (I) :

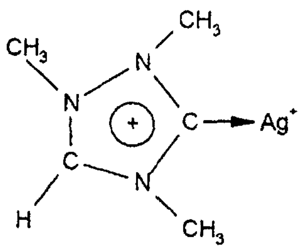

(Ia)

2 $CF_3SO_3^-$

On ajoute 0,4 g (2,4 mmol) d'acétate d'Ag à une suspension de 1 g de composé dicationique (IIIa), préparé dans l'exemple 1, dans 20 ml de tétrahydrofuranne (THF). On agite pendant 2 h à reflux. Il se forme une huile brune non miscible au THF, que l'on lave plusieurs fois avec 10 ml de THF. Après évaporation du solvant, on obtient un solide marron sensible à la lumière (rendement de 82 % par rapport au composé dicationique (IIIa)).
Ce complexe présente les caractéristiques suivantes :

- RMN $^1H$ ($CD_3CN$) : $\delta$ 4,01 (s, 3 H, $NCH_3$), 4,19 (s, 3 H, $C\underline{H}_3N\text{-}NCH_3$), 4,22 (s, 3 H, $CH_3N\text{-}NC\underline{H}_3$), 9,72 (s, 1 H, CH).
- RMN $^{13}C$ ($CD_3CN$) : $\delta$ 37,2 ($NCH_3$), 38,5 ($NCH_3$), 38,6 ($NCH_3$), 121,1 (q, $^1J_{CF}$ = 320,1 Hz, $CF_3$), 144,8 (CH) ; 189,2 (CAg).

EXEMPLE 8. Synthèse du composé (Ib) de Hg (II) :

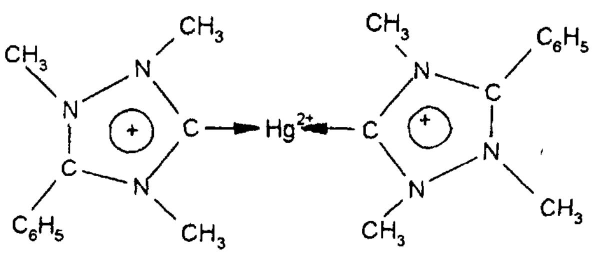

4 $CF_3SO_3^-$

On ajoute 0,16 g de diacétate de Hg (qui a été préalablement séché sous vide pendant 12 h à 50°C) et 1 ml de diméthylsulfoxyde (DMSO) à une suspension de 0,43 g de composé dicationique (IIIe), préparé dans l'exemple 5, dans 20 ml de tétrahydrofuranne (THF). On agite pendant 24 h à température ambiante. Il se forme un précipité brun soluble uniquement dans les solvants très polaires. Ce solide est lavé plusieurs fois avec du THF pour éliminer toutes traces d'acide acétique. Le complexe ainsi obtenu est recristallisé par lente diffusion d'éther dans une solution méthanolique dudit complexe. Le complexe (Ib) recristallisé présente un point de fusion de 85,4-87°C. On obtient un rendement de 86 % par rapport au composé dicationique (IIIe).
Ce complexe présente les caractéristiques suivantes :

- RMN $^1H$ ($CD_3OD$) : $\delta$ 4,70 (s, 12 H, $NC\underline{H}_3$, $^4J_{H\text{-}Hg}$ = 12,8), 4,29 (s, 6 H, $CH_3N$, $^4J_{H\text{-}Hg}$ = 8,5 Hz), 7,9-8,2 (massif, 10 H, H phényl).
- RMN $^{13}C$ ($CD_3OD$) : $\delta$ 38,79 ($CH_3N\text{-}NCH_3$), 41,75 ($NCH_3$), 121,1 (q, $^1J_{CF}$ = 320,1 Hz, $CF_3$), 156,88 ($\underline{C}$-Ph, $(^3J+^2J)_{C\text{-}Hg}$ = 86 Hz), 183,78 (CHg, $J^1_{CHg}$ = 3275 Hz).

Sa structure a également été établie par diffraction des rayons X.

EXEMPLE 9. Synthèse du composé (Ic) de Ni (II)

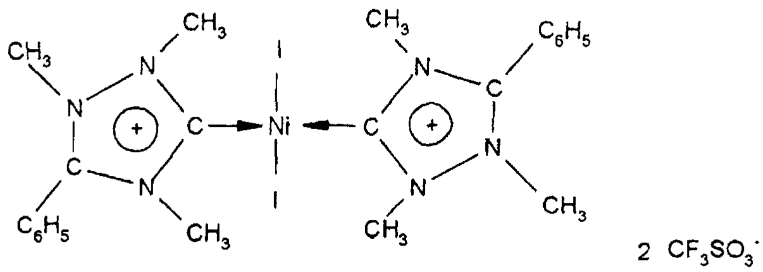

On ajoute 2,4 g de composé dicationique (IIIe), préparé dans l'exemple 5, à une suspension de 0,442 g de diacétate de Ni et 0,75 g d'iodure de Na dans 50 ml de THF. On agite pendant 2 h à reflux. Il se forme un précipité coloré qui est filtré et lavé plusieurs fois avec du THF. Le complexe ainsi obtenu est recristallisé dans un mélange acétone/éther à -30°C. Le complexe (Ic) recristallisé est rouge et il présente un point de fusion de 310,7°C (décomposition : les cristaux deviennent noirs). On obtient un rendement de 85 % par rapport au composé dicationique (IIIe).

Ce complexe présente les caractéristiques suivantes :

- RMN $^1H$ (Acétone-$d_6$) : δ 7,8-8,0 (large, 10 H, H phényl), 4,88-4,84 (2 s, 6 H, $NCH_3$), 4,54-4,49 (2 s, 6 H, $CH_3N$), 4,42-4,41 (2 s, 6 H, $CH_3N$).
- RMN $^{13}C$ ($CD_3OD$) : δ 37,0 ($CH_3N$), 37,9 ($CH_3N$), 38,4 ($CH_3N$), 121,1 (q, $^1J_{CF}$ = 320,1 Hz, $CF_3$), 130,5-130,8-134,8 (C phényl), 153,4 (<u>C</u>-Ph), 188,2 (C-Ni).

Sa structure a également été établie par diffraction des rayons X.

EXEMPLE 10. Synthèse du composé (Id) de Ni (II) :

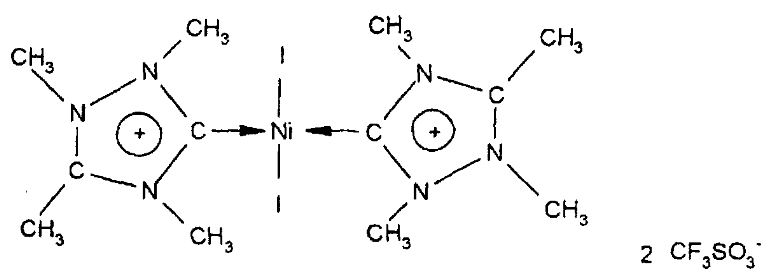

On ajoute 0,425 g de composé dicationique (IIIf), préparé dans l'exemple 6, à une suspension de 0,090 g de diacétate de Ni et 0,15 g d'iodure de Na dans 25 ml de THF. On agite pendant 2 h à reflux. Il se forme un précipité coloré qui est filtré et lavé plusieurs fois avec du THF. Le complexe ainsi obtenu est recristallisé dans un mélange acétone/éther à -30°C. Le complexe (Id) recristallisé est rouge et il présente un point de fusion de 300-305°C (décomposition : les cristaux deviennent noirs). On obtient un rendement de 79 % par rapport au composé dicationique (IIIf).

Ce complexe présente les caractéristiques suivantes :

- RMN $^1H$ (Acétone-$d_6$) : δ 4,88-4,84 (2 s, 6 H, $NCH_3$), 4,54-4,49 (2 s, 6 H, $CH_3N$), 4,42-4,41 (2 s, 6 H, $CH_3N$), 2,63 (s, 6 H, C-C$\underline{H_3}$).
- RMN $^{13}C$ ($CD_3OD$) : δ 13,86 (C-C$\underline{H_3}$), 36,1 ($CH_3N$), 37,6 ($CH_3N$), 38,5 ($CH_3N$), 121,1 (q, $^1J_{CF}$ = 320,1 Hz, $CF_3$), 154,8 (<u>C</u>-Me), 187,6 (C-Ni).

Sa structure a également été établie par diffraction des rayons X.

EXEMPLE 11. Synthèse du composé (le) de Ni (II) :

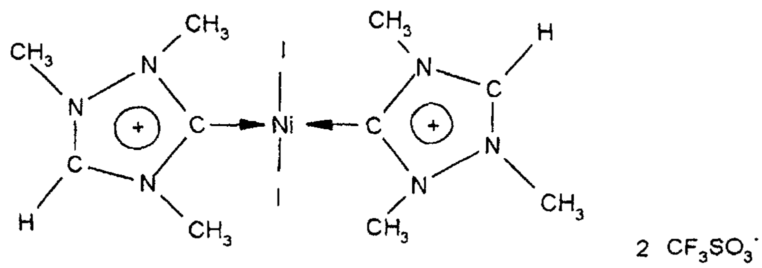

On ajoute 0,935 g de composé dicationique (IIIa), préparé dans l'exemple 1, à une suspension de 0,200 g de diacétate de Ni et 0,34 g d'iodure de Na dans 30 ml de THF. On agite pendant 2 h à reflux. il se forme un précipité coloré qui est filtré et lavé plusieurs fois avec du THF. Le complexe ainsi obtenu est recristallisé par lente diffusion d'éther dans une solution dudit complexe dans l'acétonitrile. Recristallisation dans un mélange acétone/éther à -30°C. Le complexe (le) recristallisé est rouge. On obtient un rendement de 70 % par rapport au composé dicationique (IIIa).

Ce complexe présente les caractéristiques suivantes :

- RMN $^1H$ (Acétone-$d_6$) : δ 4,38-4,35 (2 s, 6 H, $CH_3N$), 4,68-4,65 (2 s, 6 H, $CH_3N$), 4,99-4,96 (2 s, 6 H, $NCH_3$), 9,32 (s, 2 H, C<u>H</u>).

EXEMPLE 12. Synthèse du composé (If) de Pd (II) :

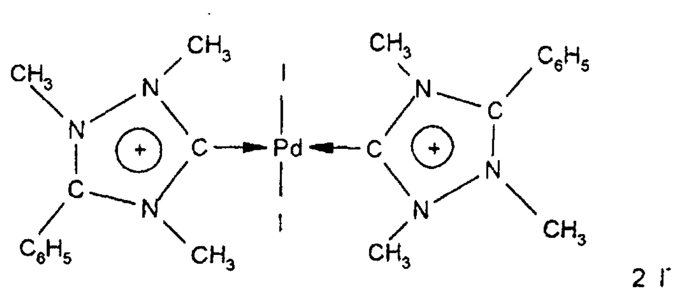

On ajoute 0,472 g de composé dicationique (IIIe), préparé dans l'exemple 5, à une suspension de 0,112 g de diacétate de Pd et 0,30 g d'iodure de Na dans 60 ml de THF. On agite pendant 2 h à reflux. Il se forme un précipité coloré qui est filtré et lavé plusieurs fois avec de l'éther. Le complexe ainsi obtenu est recristallisé à -30°C dans un mélange acétone/éther. Il se présente sous forme de cristaux jaunes. Le complexe (If) recristallisé présente un point de fusion de 211-213°C (décomposition : les cristaux deviennent marron). Au cours de la réaction, tous les anions trifluorométhylesulfonate ont été substitués par des anions iodure. On obtient un rendement de 51 % par rapport au composé dicationique (IIIe).

Ce complexe présente les caractéristiques suivantes :

- RMN $^1H$ (DMSO-$d_6$) : δ 7,8-8,1 (large, 10 H, H phényl), 4,49-4,53 (2 s, 6 H, $NCH_3$), 4,21 (s, 6 H, $CH_3N$), 4,10-4,13 (2 s, 6 H, $CH_3N$).
- RMN $^{13}C$ (DMSO-$d_6$) : δ 37,2 ($CH_3N$), 38,3 ($CH_3N$), 39,9 ($CH_3N$), 130,1-130,9-134,5 (C phényl), 152,4 (<u>C</u>-Ph), 174,5 (C-Pd).

Sa structure a également été établie par diffraction des rayons X.

EXEMPLE 13. Synthèse du composé (Ig) de Pd (II) :

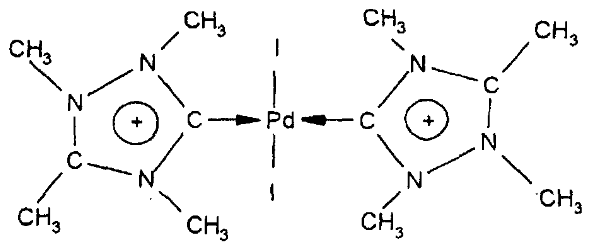

2 $I^-$

On ajoute 0,425 g de composé dicationique (IIIf), préparé dans l'exemple 6, à une suspension de 0,112 g de diacétate de Pd et 0,30 g d'iodure de Na dans 60 ml de THF. On agite pendant 2 h à reflux. Il se forme un précipité coloré qui est filtré et lavé plusieurs fois avec de l'éther. Le complexe ainsi obtenu est recristallisé à -30°C dans un mélange acétone/éther. Il se présente sous forme de cristaux rouges. Le complexe (Ig) recristallisé présente un point de fusion de 206-212°C (décomposition : les cristaux deviennent marron). Au cours de la réaction, tous les anions trifluorométhylesulfonate ont été substitués par des anions iodure. On obtient un rendement de 60 % par rapport au composé dicationique (IIIf).

Ce complexe présente les caractéristiques suivantes :

- RMN $^1H$ (DMSO-$d_6$) : δ 4,41-4,40 (2 s, 6 H, $NCH_3$), 4,29 (s, 6 H, $CH_3N$), 4,16-4,15 (2 s, 6 H, $CH_3N$), 2,95 (6 H, C-$CH_3$).

EXEMPLE 14. Synthèse du composé (Ih) de Ni (0) :

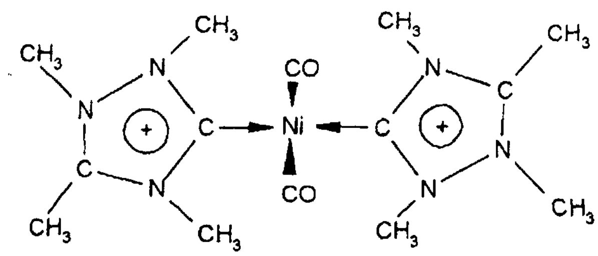

2 $CF_3SO_3^-$

Méthode A de synthèse

On dilue 1 ml de nickel tétracarbonyle dans 40 ml d'acétonitrile anhydre et dégazé. On ajoute à la solution obtenue 380 µl de pyridine et 660 µl de triéthylamine.

Une solution de 2,0 g de composé dicationique (IIIf), préparé dans l'exemple 6, dans 60 ml d'acétonitrile anhydre et dégazé est ajoutée goutte à goutte grâce à une ampoule. Un dégagement gazeux est observé et la solution se colore en jaune. Lorsque toute la solution a été introduite, on maintient encore l'agitation pendant 1 h. Le volume de la solution réactionnelle est ensuite réduit des deux tiers par évaporation sous vide du solvant, puis on rajoute 200 ml d'éther dégazé. Il se forme alors un précipité du complexe (In) qui est filtré et lavé plusieurs fois avec de l'éther. Le complexe ainsi obtenu est recristallisé dans un mélange acétone/éther à -30°C. Le complexe (Ih) se présente sous forme de cristaux jaunes. Son point de fusion n'a pas pu être déterminé précisément (décomposition : les cristaux deviennent marron). On obtient un rendement de 26 % par rapport au composé dicationique (IIIf).

Ce complexe présente les caractéristiques suivantes :

- RMN $^1H$ (DMSO-$d_6$) : δ 4,00 (s, 6 H, $NCH_3$), 3,99 (s, 6 H, $CH_3N$), 3,78 (s, 6 H, $CH_3N$), 2,72 (6 H, C-C$\underline{H}_3$).

Par spectrométrie Infra-rouge, on observe des bandes à 1982 $cm^{-1}$ et 1906 $cm^{-1}$ (CO).
Sa structure a également été établie par diffraction des rayons X.
Par analyse élémentaire on a trouvé :

- 28,99 % de C (théorie 28,89 %)
- 3,36 % de H (théorie 3,63 %)
- 12,39 % de N (théorie 12,62 %).

Méthode B de synthèse

A une solution de 0,2 g du complexe organométallique (Id) préparé dans l'exemple 10, dans 10 ml d'acétonitrile anhydre et dégazé, on ajoute 150 mg de poudre de Zn métallique activé (10 équivalents). La solution est placée sous atmosphère de monoxyde de carbone. La réaction commence en même temps que l'agitation est mise en route. La solution devient jaune. La solution est filtrée à l'aide d'une cannule, puis elle est traitée de la manière décrite pour la méthode A (à partir de l'addition d'éther pour précipiter le complexe organométallique). Le rendement obtenu est de 48 % par rapport au complexe organométallique (Id) engagé. On effectue les mêmes analyses et caractérisations que dans la méthode A et les résultats sont les mêmes.

EXEMPLE 15. Synthèse du composé (Ij) de Rh (I) :

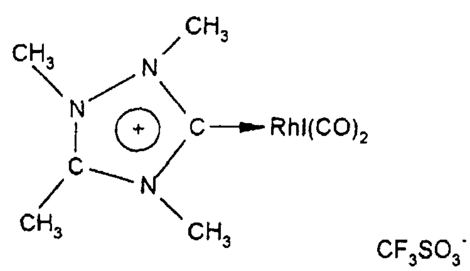

(Ij)

On ajoute une solution de 0,277 g d'acétylacétonate de di(carbonyl)-rhodium dans 10 ml de THF, à une suspension de 0,456 g de composé dicationique (IIIf), préparé dans l'exemple 6, et de 0,16 g de NaI dans 40 ml de THF. On agite pendant 2 jours à 50°C. On récupère un solide brun après évaporation du solvant et plusieurs lavages à l'éther. Le rendement est de 70 % par rapport au composé dicationique (IIIf).
Ce complexe présente les caractéristiques suivantes :

- RMN $^1H$ (DMSO-$d_6$) : δ 4,47 (s, 3 H, $NCH_3$), 4,28 (s, 3 H, $CH_3N$), 4,20 (s, 3 H, $CH_3N$), 2,96 (s, 3 H, C-$CH_3$).
- RMN $^{13}C$ (DMSO-$d_6$) : δ 10,5 (s, C-$\underline{C}H_3$), 35,2 (s, $NCH_3$), 36,8 (s, $CH_3N$), 37,9 (s, $CH_3N$), 154,2 (s, $\underline{C}$-$CH_3$), 183,0 (d, $^1J$(Rh-C) = 82,9 Hz, CO) , 188,1 (d, $^1J$(Rh-C) = 42,2 Hz, C carbène).

En Infra-rouge, on observe une bande à 1975 $cm^{-1}$ (CO).

EXEMPLE 16. Synthèse du composé (Ik) de Rh (I) :

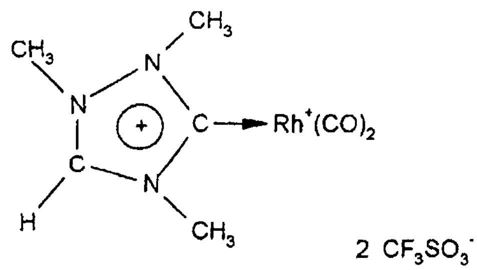

(Ik)

On ajoute une solution de 0,200 g d'acétylacétonate de di(carbonyl)-rhodium dans 10 ml de THF, à une suspension de 0,16 g de composé dicationique (IIIa), préparé dans l'exemple 1 dans 10 ml de THF. On agite pendant 15 h à 50°C. Il se forme un précipité solide brun qui est lavé plusieurs fois à l'éther. Le rendement en complexe (Ik) est de 67 % par rapport au composé dicationique (IIIa).

Ce complexe présente les caractéristiques suivantes :

- RMN $^1H$ (DMSO-$d_6$) : δ 4,20 (s, 3 H, $NCH_3$), 4,22 (s, 3 H, $CH_3N$), 4,25 (s, 3 H, $CH_3N$), 9,66 (s, 1 H, CH).
- RMN $^{13}C$ (DMSO-$d_6$) : δ 38,6 (s, $NCH_3$), 38,7 (s, $NCH_3$), 39,1 (s, $CH_3N$), 37,9 (s, $CH_3N$), 121,1 (q, $^1J_{CF}$ = 320,1 Hz, $CF_3$), 145,5 (s, CH), 186,1 (d, $^1J$(Rh-C) = 76 Hz, CO), 187,9 (d, $^1J$(Rh-C) = 43,3 Hz, C carbène).

En Infra-rouge, on observe des bandes à 2029 (i) $cm^{-1}$ et 1985 (m) $cm^{-1}$ (CO).

EXEMPLE 17. Catalyse de l'hydroformylation du styrène par le composé (Ij) de Rh (I)

La réaction d'hydroformylation du styrène est effectuée dans un réacteur en acier inoxydable de 100 ml, comportant une double enveloppe permettant le chauffage par circulation d'huile, à une température contrôlée à l'aide d'un thermostat. Le couvercle du réacteur est équipé d'une vanne d'introduction ou d'évacuation des gaz sous pression, d'un manomètre de précision, d'une soupape de sécurité et d'une vanne à bille permettant le transfert de solutions avec une seringue. Un pôt en Téflon® est ajusté à l'intérieur de l'autoclave et contient un barreau aimanté pour l'agitation des réactifs.

Le complexe (Ij) de Rh (I) préparé dans l'exemple 15 est chargé (40 mg) dans le pôt en Téflon® ainsi que 100 mg de triphénylphosphine (5 équivalents par rapport au catalyseur). L'autoclave est assemblé et purgé trois fois à l'argon. On charge ensuite 15 ml de tétrahydrofuranne, 5 ml de styrène et 60 mg de triéthylamine (4 équivalents par rapport au catalyseur). On établit alors une pression de monoxyde de carbone (15 bar) et d'hydrogène (15 bar). On chauffe à 60°C sous agitation magnétique. Après 4 h de réaction en température, l'autoclave est rapidement refroidi dans un mélange acétone/azote liquide et on effectue la dépressurisation en une heure. On analyse par chromatographie en phase vapeur (CPV) un échantillon du mélange réactionnel.

On obtient les résultats suivants :

- taux de conversion du styrène : 95 %
- sélectivité en aldéhydes : 97 %
- rapport aldéhyde branché/aldéhyde linéaire : 95/5
- fréquence de rotation du catalyseur : 75 $h^{-1}$ (calculée en moyenne sur le temps de réaction en prenant un coefficient de réponse en CPV identique pour tous les produits).

EXEMPLE 18. Catalyse de l'hydroformylation du styrène par le composé (Ik) de Rh (I)

On répète l'exemple 17 avec 40 mg de complexe Ik) préparé dans l'exemple 16 et sans mettre en oeuvre de triphénylphosphine.

Après 3 h de réaction à 80°C, l'autoclave est rapidement refroidi dans un mélange acétone/azote liquide et on effectue la dépressurisation en une heure. On analyse par chromatographie en phase vapeur (CPV) un échantillon du mélange réactionnel.

On obtient les résultats suivants :

- taux de conversion du styrène : 96 %
- sélectivité en aldéhydes : 100 %
- rapport aldéhyde branché/aldéhyde linéaire : 25/75
- fréquence de rotation du catalyseur : 79 $h^{-1}$ (calculée en moyenne sur le temps de réaction en prenant un coefficient de réponse en CPV identique pour tous les produits).

## Revendications

**1.** Complexes organométalliques comprenant des carbènes hétérocycliques. **caractérisés en ce qu'**ils répondent à la formule générale (I)

$$[(Z^+ X^-)_m M L_n] Y \qquad (I)$$

dans laquelle :

- $Z^+$ représente un ion 1,2,4-triazolium-5-ylidène dont au moins une partie des atomes du cycle sont substitués par des radicaux hydrocarbonés
- L représente un ligand qui peut être ionique ou neutre
- M représente un métal choisi parmi les éléments de transition des groupes 1b, 2b, 3b, 4b, 5b, 6b, 7b et 8 de la Classification périodique des éléments
- $X^-$ représente un anion organique ou minéral
- m représente un nombre entier de 1 à 6
- n représente un nombre entier de 0 à 5
- la somme de m et de n est égale ou inférieure à 6
- Y représente un anion ou un cation tel que le complexe métallique soit électriquement neutre.

**2.** Complexes organométalliques selon la revendication 1, **caractérisés en ce que** les ions 1,2,4-triazolium-5-ylidéne $Z^+$ répondent à la formule générale (II) :

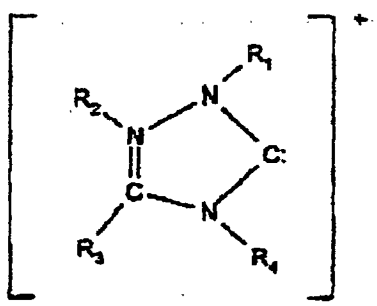

(II)

dans laquelle $R_1$, $R_2$, $R_3$ et $R_4$, identiques ou différents, représentent :

- un radical alkyle, linéaire ou ramifié, un radical cycloalkyle,
- un radical aryle,
- un radical alkyle qui comprend un ou plusieurs substituants tels qu'un radical aryle, un radical alcoxy, un atome d'halogène, un groupe hydrophile comme :
    - $COOM_1$, $-SO_3M_1$, $-PO_3M_1$, $M_1$ représentant un reste cationique minéral ou organique choisi parmi le proton, les cations dérivés des métaux alcalins ou alcalino-terreux, les cations ammonium $-N(R)_4$ dons la formule desquels les symboles R, identiques ou différents, représentent un atome d'hydrogène ou un radical alkyle ayant de 1 à 4 atomes de carbone,

- $N(R)_3$ $Y_a$ dans la formule duquel les symboles R, identiques ou différents, représentent un atome d'hydrogène ou un radical alkyle ayant de 1 à 12 atomes de carbone, et $Y_a$ représente un anion minéral ou organique,
- OH

- un radical aryle ou aralkyle ou cycloalkyle qui comprend sur le cycle un ou plusieurs substituants tels qu'un radical alkyle, un radical alcoxy, un atome d'halogène, un groupe hydrophile comme :

  - $COOM_1$, $-SO_3M_1$, $-PO_3M_1$, $M_1$ représentant un reste cationique minéral ou organique choisi parmi le proton, les cations dérivés des métaux alcalins ou alcalino-terreux, les cations ammonium $-N(R)_4$ dans la formula desquels les symboles R, identiques ou différents, représentent un atome d'hydrogène ou un radical alkyle ayant de 1 à 12 atomes de carbone,
  - $N(R)_3$ $Y_a$ dans la formule duquel les symboles R, identiques ou différents, représentent un atome d'hydrogène ou un radical alkyle ayant de 1 à 12 atomes de carbone, et $Y_a$ représente un anion minéral ou organique,
  - OH

$R_3$ représente également un atome d'hydrogène
$R_1$ et/ou $R_4$ peuvent aussi représenter chacun un reste ligand organique lié à l'atome d'azote de l'ion 1,2,4-triazolium-5-ylidène par un radical alkylène ou arylène, ledit reste ligand pouvant être un reste phosphite, un reste phosphonite, un reste phosphinite,
un reste phosphine ou un reste amine tertiaire aliphatique, cycloaliphatique, aromatique ou hétérocyclique et servant de ligand L au métal M.

3. Complexes organométalliques selon l'une des revendications 1 ou 2, **caractérisés en ce que** $R_1$ et/ou $R_4$ représentent un reste ligand organique lié à l'atome d'azote de l'ion 1,2,4-triazolium-5-ylidène par un radical alkylène ou phénylène, ledit reste ligand dérivant de phosphates de phényle ou d'alkyle, substitués ou non par des substituants tels que définis précédemment, ou de phosphonites de phényle ou d'alkyle, substitués ou non par des substituants tels que définis précédemment, ou de phosphinites de phényle ou d'alkyle, substitués ou non par des substituants tels que définis précédemment, ou de phénylphosphines ou d'alkylphosphines, substituées ou non par des substituants tels que définis précédemment.

4. Complexes organométalliques selon l'une des revendications 1 à 3, **caractérisés en ce que** L représente un ligand ionique choisi parmi les halogénures, les cyanures ou un ligand neutre choisi parmi le monoxyde de carbone, un isonitrile, une phosphine, un phosphite organique, un phosphonate ou un phosphonite.

5. Complexes organométalliques selon l'une des revendications 1 à 4, **caractérisés en ce que** M représente un métal choisi parmi le nickel, le cobalt, le fer, le ruthénium, le rhodium, le palladium, l'osmium, l'iridium, le platine, le cuivre, l'argent, l'or, le zinc, le cadmium, le mercure.

6. Complexes organométalliques selon l'une des revendications 1 à 5, **caractérisés en ce que** les anions $X^-$ sont des anions faiblement coordinants et constituent majoritairement les contre-ions des ions 1,2,4-triazolium-5-ylidène.

7. Complexes organométalliques selon la revendication 6, **caractérisés en ce que** les anions faiblement coordinants $X^-$ sont choisis parmi le trifluorométhylsulfonate, le tétrafluoroborate, l'hexafluorophosphate, le tétrachlorure d'aluminium, le tétrabromure d'aluminium, le tétrafluorure d'aluminium, le tétraiodure d'aluminium, le tétrachlorure de gallium, le tétrabromure de gallium, le tétrafluorure de gallium, le tétraiodure de gallium.

8. Procédé de préparation des complexes organométalliques de formule générale (I), **caractérisé en ce que** l'on fait réagir un composé hétérocyclique dicationique de formule générale (III) :

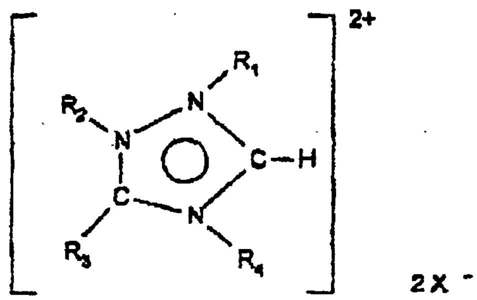

(III)

dans laquelle

- les symboles $R_1$, $R_2$, $R_3$ et $R_4$ ont les significations indiquées précédemment pour les ions 1,2,4-triazolium-5-ylidène de formule (II)
- $X^-$ représente un anion tel que ceux indiqués pour la formule générale (I).

avec un composé du métal M en présence d'une base minérale ou organique.

9. Procédé de préparation selon la revendication 8, **caractérisé en ce que** la réaction est en milieu liquide, en ajoutant le composé du métal M et le cas échéant la base minérale ou organique à la solution ou à la suspension du composé (III), ou en opérant avec un autre ordre d'introduction des réactifs, à température ambiante ou de préférence à une température de 25°C à 150°C.

10. Utilisation des complexes organométalliques de formule (I) selon l'une des revendications 1 à 7, comme catalyseurs de réactions chimiques.

11. Utilisation selon la revendication 10 pour l'hydrosilylation d'alcènes ou d'alcynes en particulier en présence de complexes du ruthénium, l'hydrosilylation des cétones en présence de complexes du ruthénium ou du rhodium, la réaction de Heck en présence de complexes du palladium, l'hydrogénation des oléfines, des aldéhydes, des acides, des énamides et des composés nitroaromatiques en présence de complexes du ruthénium, du rhodium, du platine ou du palladium, l'hydroformylation et l'hydrocarbonylation des oléfines en présence de complexes du rhodium, l'hydrocyanation des oléfines en présence de complexes du nickel, la synthèse du furanne en présence de complexes du ruthénium, la métathèse des oléfines en présence de complexes du ruthénium, la polymérisation des acrylates en présence de complexes du nickel.

## Claims

1. Organometallic complexes comprising heterocyclic carbenes, **characterized in that** they correspond to the general formula (I)

$$[(Z^+X^-)_m ML_n]Y \quad (I)$$

in which:

- $Z^+$ represents a 1,2,4-triazolium-5-ylidene ion, at least a portion of the atoms of the ring of which are substituted by hydrocarbon-comprising radicals,
- L represents a ligand, which can be ionic or neutral,
- M represents a metal chosen from the transition elements from Groups 1b, 2b, 3b, 4b, 5b, 6b, 7b and 8 of the Periodic Classification of the Elements,
- $X^-$ represents an organic or inorganic anion,
- m represents an integer from 1 to 6,
- n represents an integer from 0 to 5,
- the sum of m and of n is equal to or less than 6,

- Y represents an anion or a cation such that the metal complex is electrically neutral.

**2.** Organometallic complexes according to Claim 1, **characterized in that** the 1,2,4-triazolium-5-ylidene ions $Z^+$ correspond to the general formula (II):

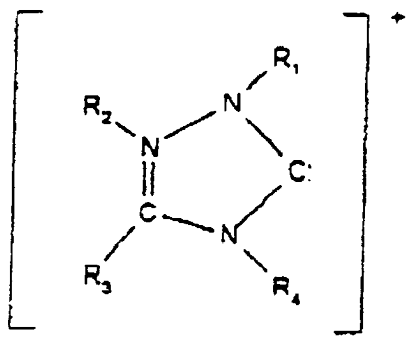

(II)

in which $R_1$, $R_2$, $R_3$ and $R_4$, which are identical or different, represent:

- a linear or branched alkyl radical or a cycloalkyl radical,
- an aryl radical,
- an alkyl radical which comprises one or more substituents, such as an aryl radical, an alkoxy radical, a halogen atom or a hydrophilic group, for example:

  - $COOM_1$, $-SO_3M_1$ or $-PO_3M_1$, $M_1$ representing an inorganic or organic cationic residue chosen from a proton, cations derived from alkali metals or alkaline earth metals, or ammonium cations $-N(R)_4$, in the formula of which the R symbols, which are identical or different, represent a hydrogen atom or an alkyl radical having from 1 to 4 carbon atoms,
  - $N(R)_3Y_a$, in the formula of which the R symbols, which are identical or different, represent a hydrogen atom or an alkyl radical having from 1 to 12 carbon atoms and $Y_a$ represents an inorganic or organic anion,
  - OH,

- an aryl or aralkyl or cycloalkyl radical which comprises, on the ring, one or more substituents, such as an alkyl radical, an alkoxy radical, a halogen atom or a hydrophilic group, for example:
- $COOM_1$, $-SO_3M_1$ or $-PO_3M_1$, $M_1$ representing an inorganic or organic cationic residue chosen from a proton, cations derived from alkali metals or alkaline earth metals, or ammonium cations $-N(R)_4$, in the formula of which cations the R symbols, which are identical or different, represent a hydrogen atom or an alkyl radical having from 1 to 12 carbon atoms,
- $N(R)_3Y_a$, in the formula of which the R symbols, which are identical or different, represent a hydrogen atom or an alkyl radical having from 1 to 12 carbon atoms and $Y_a$ represents an inorganic or organic anion,
- OH,

$R_3$ also represents a hydrogen atom,
$R_1$ and/or $R_4$ can also each represent an organic ligand residue bonded to the nitrogen atom of the 1,2,4-triazolium-5-ylidene ion via an alkylene or arylene radical, it being possible for the said ligand residue to be a phosphite residue, a phosphonite residue, a phosphinite residue, a phosphine residue or an amine residue which is tertiary and aliphatic, cycloaliphatic, aromatic or heterocyclic and which acts as ligand L to the metal M.

**3.** Organometallic complexes according to either of Claims 1 and 2, **characterized in that** $R_1$ and/or $R_4$ represent an organic ligand residue bonded to the nitrogen atom of the 1,2,4-triazolium-5-ylidene ion via an alkylene or phenylene radical, the said ligand residue deriving from phenyl or alkyl phosphites, substituted or unsubstituted by substituents as defined above, or from phenyl or alkyl phosphonites, substituted or unsubstituted by substituents as defined above, or from phenyl or alkyl phosphinites, substituted or unsubstituted by substituents as defined above, or from phenylphosphines or alkylphosphines, substituted or unsubstituted by substituents as defined above.

**4.** Organometallic complexes according to one of Claims 1 to 3, **characterized in that** L represents an ionic ligand

chosen from halides or cyanides or a neutral ligand chosen from carbon monoxide, an isonitrile, a phosphine, an organic phosphite, a phosphonate or a phosphonite.

**5.** Organometallic complexes according to one of Claims 1 to 4, **characterized in that** M represents a metal chosen from nickel, cobalt, iron, ruthenium, rhodium, palladium, osmium, iridium, platinum, copper, silver, gold, zinc, cadmium or mercury.

**6.** Organometallic complexes according to one of Claims 1 to 5, **characterized in that** the $X^-$ anions are weakly coordinating anions and predominantly constitute the counterions of the 1,2,4-triazolium-5-ylidene ions.

**7.** Organometallic complexes according to Claim 6, **characterized in that** the weakly coordinating anions $X^-$ are chosen from trifluoromethanesulphonate, tetrafluoroborate, hexafluorophosphate, aluminium tetrachloride, aluminium tetrabromide, aluminium tetrafluoride, aluminium tetraiodide, gallium tetrachloride, gallium tetrabromide, gallium tetrafluoride or gallium tetraiodide.

**8.** Process for the preparation of the organometallic complexes of general formula (I), **characterized in that** a dicationic heterocyclic compound of general formula (III):

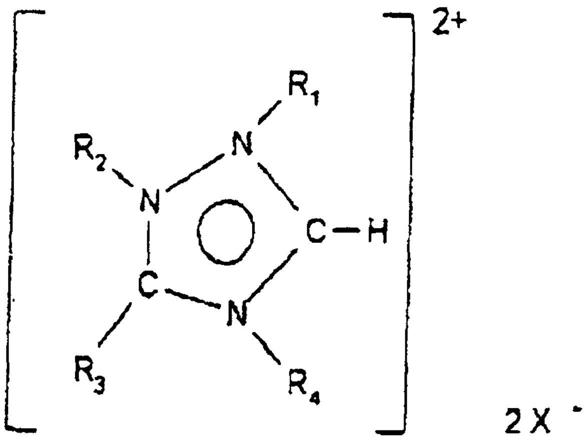

(III)

in which

- the $R_1$, $R_2$, $R_3$ and $R_4$ symbols have the meanings shown above for the 1,2,4-triazolium-5-ylidene ions of formula (II),
- $X^-$ represents an anion such as those shown for the general formula (I),
  is reacted with a compound of the metal M in the presence of an inorganic or organic base.

**9.** Preparation process according to Claim 8, **characterized in that** the reaction is in liquid medium, the compound of the metal M and, if appropriate, the inorganic or organic base being added to the solution or suspension of the compound (III) or the preparation being carried out with another order of introduction of the reactants, at room temperature or, preferably, at a temperature of 25°C to 150°C.

**10.** Use of the organometallic complexes of formula (I) according to one of Claims 1 to 7, as catalysts of chemical reactions.

**11.** Use according to Claim 10 in the hydrosilylation of alkenes or of alkynes, in particular in the presence of ruthenium complexes, the hydrosilylation of ketones in the presence of ruthenium or rhodium complexes, the Heck reaction in the presence of palladium complexes, the hydrogenation of olefins, aldehydes, acids, enamides and nitroaromatic compounds in the presence of ruthenium, rhodium, platinum or palladium complexes, the hydroformylation and the hydrocarbonylation of olefins in the presence of rhodium complexes, the hydrocyanation of olefins in the presence of nickel complexes, the synthesis of furan in the presence of ruthenium complexes, the metathesis of olefins in the presence of ruthenium complexes, or the polymerization of acrylates in the presence of nickel complexes.

**Patentansprüche**

1. Organometallkomplexe, umfassend heterocyclische Carbene, **dadurch gekennzeichnet, daß** sie der allgemeinen Formel (I)

$$[(Z^+ X^-)_m M L_n]Y \qquad (I)$$

entsprechen, in der

- $Z^+$ ein Ion 1,2,4-Triazolium-5-yliden darstellt, bei dem mindestens ein Teil der Atome des Ringes durch KohlenwasserstoffReste substituiert ist,
- L einen Liganden bedeutet, der ionisch oder neutral sein kann,
- M ein Metall darstellt, ausgewählt unter den Übergangselementen der Gruppen 1b, 2b, 3b, 4b, 5b, 6b, 7b und 8 des Periodensystems der Elemente,
- $X^-$ ein mineralisches oder organisches Anion ist,
- m eine ganze Zahl von 1 bis 6 ist,
- n eine ganze Zahl von 0 bis 5 ist,
- die Summe von m und n gleich oder niedriger als 6 ist,
- Y ein solches Anion oder Kation darstellt, daß der Metallkomplex elektrisch neutral ist.

2. Organometallkomplexe nach Anspruch 1, **dadurch gekennzeichnet, daß** die Ionen 1,2,4-Triazolium-5-yliden $Z^+$ der allgemeinen Formel (II)

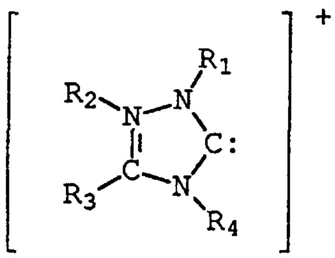

(II)

entsprechen, in der
$R_1$, $R_2$, $R_3$ und $R_4$, gleich oder verschieden, darstellen:

- einen linearen oder verzweigten Rest Alkyl, einen Rest Cycloalkyl,
- einen Rest Aryl,
- einen Rest Alkyl, der einen oder mehrere Substituenten umfaßt wie einen Rest Aryl, einen Rest Alkoxy, ein Halogenatom, eine hydrophile Gruppe wie:
  - $COOM_1$, $-SO_3M_1$, $-PO_3M_1$, worin $M_1$ ein mineralischer oder organischer kationischer Rest ist, ausgewählt unter dem Proton, den von Alkalimetallen oder Erdalkalimetallen abgeleiteten Kationen, den Ammonium-Kationen $-N(R)_4$, worin die Symbole R, gleich oder verschieden, ein Wasserstoffatom oder einen Rest Alkyl mit 1 bis 4 Kohlenstoffatomen darstellen,
  - $N(R)_3Y_a$, worin die Symbole R, gleich oder verschieden, ein Wasserstoffatom oder einen Rest Alkyl mit 1 bis 12 Kohlenstoffatomen darstellen und $Y_a$ ein mineralisches oder organisches Anion ist,
  - OH,
- einen Rest Aryl oder Aralkyl oder Cycloalkyl, der am Ring einen oder mehrere Substituenten umfaßt, wie einen Rest Alkyl, einen Rest Alkoxy, ein Halogenatom, eine hydrophile Gruppe wie:
  - $COOM_1$, $-SO_3M_1$, $-PO_3M_1$, worin $M_1$ ein mineralischer oder organischer kationischer Rest ist, ausgewählt unter dem Proton, den von Alkalimetallen oder Erdalkalimetallen abgeleiteten Kationen, den Ammonium-Kationen $-N(R)_4$, worin die Symbole R, gleich oder verschieden, ein Wasserstoffatom oder einen Rest Alkyl mit 1 bis 12 Kohlenstoffatomen darstellen,

- $N(R)_3Y_a$, worin die Symbole R, gleich oder verschieden, ein Wasserstoffatom oder einen Rest Alkyl mit 1 bis 12 Kohlenstoffatomen darstellen und $Y_a$ ein mineralisches oder organisches Anion ist,
- OH,

$R_3$ auch ein Wasserstoffatom darstellt,
$R_1$ und/oder $R_4$ auch jeweils einen organischen Liganden-Rest bedeuten können, gebunden an das Stickstoffatom des Ions 1,2,4-Triazolium-5-yliden durch einen Rest Alkylen oder Arylen, wobei der genannte Liganden-Rest ein Phosphit-Rest, ein Phosphonit-Rest, ein Phosphinit-Rest, ein Phosphin-Rest oder ein Rest eines aliphatischen, cycloaliphatischen, aromatischen oder heterocyclischen tertiären Amins sein kann und als Ligand L zum Metall M dient.

3. Organometallkomplexe nach einem der Ansprüche 1 oder 2, **dadurch gekennzeichnet, daß** $R_1$ und/oder $R_4$ einen organischen Liganden-Rest darstellen, gebunden an das Stickstoffatom des Ions 1,2,4-Triazolium-5-yliden durch einen Rest Alkylen oder Phenylen, wobei sich der genannte Liganden-Rest von Phenylphosphiten oder von Alkylphosphiten, substituiert oder nicht substituiert durch die wie vorstehend definierten Substituenten, oder von Phenylphosphoniten oder von Alkylphosphoniten, substituiert oder nicht substituiert durch die wie vorstehend definierten Substituenten, oder von Phenylphosphiniten oder von Alkylphosphiniten, substituiert oder nicht substituiert durch die wie vorstehend definierten Substituenten, oder von Phenylphosphinen oder von Alkylphosphinen, substituiert oder nicht substituiert durch die wie vorstehend definierten Substituenten, ableitet.

4. Organometallkomplexe nach einem der Ansprüche 1 bis 3, **dadurch gekennzeichnet, daß** L einen ionischen Liganden darstellt, ausgewählt unter den Halogeniden, den Cyaniden oder den neutralen Liganden, ihrerseits ausgewählt unter Kohlenmonoxid, einem Isonitril, einem Phosphin, einem organischen Phosphit, einem Phosphonat oder einem Phosphonit.

5. Organometallkomplexe nach einem der Ansprüche 1 bis 4, **dadurch gekennzeichnet, daß** M ein Metall darstellt, ausgewählt unter Nickel, Cobalt, Eisen, Ruthenium, Rhodium, Palladium, Osmium, Iridium, Platin, Kupfer, Silber, Gold, Zink, Cadmium und Quecksilber.

6. Organometallkomplexe nach einem der Ansprüche 1 bis 5, **dadurch gekennzeichnet, daß** die Anionen $X^-$ leicht koordinierbare Anionen sind und in überwiegendem Maße die Gegenionen der Ionen 1,2,4-Triazolium-5-yliden bilden.

7. Organometallkomplexe nach Anspruch 6, **dadurch gekennzeichnet, daß** die leicht koordinierbaren Anionen $X^-$ ausgewählt werden unter Trifluormethylsulfonat, Tetrafluorborat, Hexafluorphosphat, Aluminiumtetrachlorid, Aluminiumtetrabromid, Aluminiumtetrafluorid, Aluminiumtetraiodid, Galliumtetrachlorid, Galliumtetrabromid, Galliumtetrafluorid, Galliumtetraiodid.

8. Verfahren zur Herstellung von Organometallkomplexen der allgemeinen Formel (I), **dadurch gekennzeichnet, daß** man eine dikationische heterocyclische Verbindung der allgemeinen Formel (III)

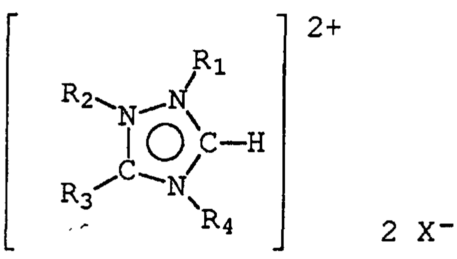

(III)

in der

- die Symbole $R_1$, $R_2$, $R_3$ und $R_4$ die vorstehend bei den Ionen 1,2,4-Triazolium-5-yliden der Formel (II) angegebenen Bedeutungen besitzen, und
- $X^-$ ein solches Anion ist, wie es bei der allgemeinen Formel (I) angegeben wurde,

mit einer Verbindung des Metalls M in Anwesenheit einer Mineralbase oder organischen Base zur Reaktion bringt.

**9.** Verfahren zur Herstellung nach Anspruch 8, **dadurch gekennzeichnet, daß** die Reaktion im flüssigen Medium erfolgt, indem man die Verbindung des Metalls M und gegebenenfalls die Mineralbase oder organische Base zu der Lösung oder der Suspension der Verbindung (III) gibt, oder indem man auch mit einer anderen Reihenfolge des Eintragens der Reaktanden arbeitet sowie bei Umgebungstemperatur oder vorzugsweise bei einer Temperatur zwischen 25 °C und 160 °C.

**10.** Verwendung der Organometallkomplexe der Formel (I) nach einem der Ansprüche 1 bis 7 als Katalysator bei chemischen Reaktionen.

**11.** Verwendung nach Anspruch 10 für die Hydrosilylierung von Alkenen oder Alkinen, insbesondere in Anwesenheit von Komplexen des Rutheniums, der Hydrosilylierung von Ketonen in Anwesenheit von Komplexen des Rutheniums oder des Rhodiums, der Reaktion nach Heck in Anwesenheit von Komplexen des Palladiums, der Hydrierung von Olefinen, Aldehyden, Säuren, Enamiden und nitroaromatischen Verbindungen in Anwesenheit von Komplexen des Rutheniums, Rhodiums, Platins oder Palladiums, der Hydroformylierung und Hydrocarbonylierung von Olefinen in Anwesenheit von Komplexen des Rhodiums, der Hydrocyanierung von Olefinen in Anwesenheit von Komplexen des Nickels, der Furan-Synthese in Anwesenheit von Komplexen des Rutheniums, der Metathese von Olefinen in Anwesenheit von Komplexen des Rutheniums, der Polymerisation von Acrylaten in Anwesenheit von Komplexen des Nickels.